(19) **Europäisches Patentamt / European Patent Office / Office européen des brevets**

(11) **EP 4 380 590 B1**

(12) **FASCICULE DE BREVET EUROPEEN**

(45) Date de publication et mention de la délivrance du brevet:
**08.10.2025 Bulletin 2025/41**

(21) Numéro de dépôt: **22757603.0**

(22) Date de dépôt: **04.08.2022**

(51) Classification Internationale des Brevets (IPC):
*A61K 36/88* (2006.01)   *A23L 33/105* (2016.01)
*A24B 15/167* (2020.01)   *A61K 31/05* (2006.01)
*A61K 31/11* (2006.01)   *A61K 31/352* (2006.01)
*A61K 36/185* (2006.01)   *A61P 25/00* (2006.01)
*A61P 25/24* (2006.01)

(52) Classification Coopérative des Brevets (CPC):
(C-Sets disponibles)
A61P 25/00; A23L 33/105; A24B 15/167;
A24B 15/283; A24B 15/303; A61K 8/33;
A61K 8/347; A61K 8/9789; A61K 8/9794;
A61K 31/01; A61K 31/05; A61K 31/11;
A61K 31/352; A61K 31/7028; A61K 31/7034;
(Cont.)

(86) Numéro de dépôt international:
**PCT/EP2022/071900**

(87) Numéro de publication internationale:
**WO 2023/012258 (09.02.2023 Gazette 2023/06)**

(54) **COMPOSITION A BASE DE CROCUS SATIVUS ET DE CANNABIS SATIVA**

ZUSAMMENSETZUNG AUF BASIS VON CROCUS SATIVUS UND CANNABIS SATIVA

COMPOSITION BASED ON CROCUS SATIVUS AND CANNABIS SATIVA

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **04.08.2021 FR 2108463**

(43) Date de publication de la demande:
**12.06.2024 Bulletin 2024/24**

(73) Titulaire: **Activ'Inside**
**33750 Beychac-et-Caillau (FR)**

(72) Inventeurs:
• **GAUDOUT, David**
  **33360 CARIGNAN DE BORDEAUX (FR)**
• **REY, Stéphane**
  **26200 MONTELIMAR (FR)**
• **LEMAIRE, Benoit**
  **33500 LIBOURNE (FR)**
• **DUBREUIL, Séverine**
  **44119 TREILLIERES (FR)**
• **MORAS, Benjamin**
  **33270 FLOIRAC (FR)**
• **BORNERIE, Mégane**
  **33600 PESSAC (FR)**
• **STANISIERE, Julien**
  **33000 BORDEAUX (FR)**
• **DEVULPILLIERES, Astrid**
  **33000 BORDEAUX (FR)**

(74) Mandataire: **Aquinov**
  **12, Cours Xavier Arnozan**
  **33000 Bordeaux (FR)**

(56) Documents cités:
**EP-A1- 3 446 678     WO-A1-2018/020013**
**WO-A1-2018/173049     WO-A1-2020/146383**

• **DATABASE TKDL [online] 1 January 1825 (1825-01-01), ANONYMOUS: "Kuhl-e- Kurkum", XP093238404, Database accession no. FA1/114B**

- DATABASE TKDL [online] 1 January 1950 (1950-01-01), ANONYMOUS: "Suddha Bhanga Visista Gunah Aur Matra", XP093238405, Database accession no. AK1/758
- JACKSON PHILIPPA A ET AL: "Effects of Saffron Extract Supplementation on Mood, Well-Being, and Response to a Psychosocial Stressor in Healthy Adults: A Randomized, Double-Blind, Parallel Group, Clinical Trial", FRONTIERS IN NUTRITION (LAUSANNE), 1 January 2020 (2020-01-01), Switzerland, pages 606124 - 606124, XP055905169, Retrieved from the Internet <URL:https://www.ncbi.nlm.nih.gov/ pmc/articles/PMC7882499/> [retrieved on 20220324], DOI: 10.3389/fnut.2020.606124
- FERBER SARI GOLDSTEIN ET AL: "The "Entourage Effect": Terpenes Couple with Cannabinoids for the Treatment of Mood Disorders and Anxiety Disorders", vol. 18, no. 2, 1 February 2020 (2020-02-01), NL, pages 87 - 96, XP055855138, ISSN: 1570-159X, Retrieved from the Internet <URL:https://www.ncbi.nlm.nih.gov/ pmc/articles/PMC7324885/pdf/CN-18-87.pdf> DOI: 10.2174/1570159X17666190903103923

(52) Classification Coopérative des Brevets (CPC):
(Cont.)**A61K 36/185; A61K 36/88; A61P 25/24; A61Q 19/00;** A61K 2800/10

C-Sets
**A61K 31/01, A61K 2300/00;
A61K 31/05, A61K 2300/00;
A61K 31/11, A61K 2300/00;
A61K 31/352, A61K 2300/00;
A61K 31/7028, A61K 2300/00;
A61K 31/7034, A61K 2300/00;
A61K 36/185, A61K 2300/00;
A61K 36/88, A61K 2300/00**

**Description**

**Domaine technique**

L'invention concerne une composition comprenant un mélange de molécules obtenu (i) d'un extrait de *Crocus sativus* comprenant au moins du safranal, et (ii) d'un extrait de *Cannabis sativa* comprenant du cannabidiol (CBD) ou d'un extrait de *Crocus sativus* et de *Cannabis sativa* et ses utilisations comme produit pharmaceutique, complément alimentaire, ou produit de nutrition, produit cosmétique, ou liquide adapté pour les cigarettes électroniques.

**Etat de l'art**

De nombreuses études cliniques ont mis en évidence l'efficacité d'extrait de safran, en particulier de *Crocus sativus* dans le traitement de la dépression, mais également sur des populations saines présentant des troubles de l'humeur dus à des conditions physiologiques particulières telles que la ménopause ou le syndrome prémenstruel.

Plus récemment une étude clinique randomisée en double aveugle a mis en évidence l'efficacité d'un extrait de safran titré à 0,2% de safranal par la méthode HPLC tel que décrit dans le brevet FR 3054443, à la dose de 30mg/ jour, dans l'amélioration de l'humeur chez des sujets sains ainsi que la diminution de la variabilité du rythme cardiaque induite par le stress aigu (Jackson Philippa et al., Effects of Saffron Extract Supplementation on Mood, Well-Being, and Response to a Psychosocial Stressor in Healthy Adults: A Randomized, Double-Blind, Parallel Group, Clinical Trial, 2021).

Ces différentes études soulignent les bénéfices du safran et l'importance du safranal sur les troubles de l'humeur, la dépression mais également la gestion du stress et de l'anxiété. Or, le stress chronique peut provoquer des effets délétères sur la santé des décennies plus tard, par exemple un risque accru de démence (Wheelan, 2018).

D'autres études cliniques ont également montré qu'une supplémentation en safran chez des sujets sains ou chez des patients atteints de déclin cognitif ou de la maladie d'Alzheimer permettait d'améliorer ou de maintenir les performances cognitives par rapport à un placebo, ou avec une efficacité équivalente à celle de médicaments de référence (Avgerinos, 2020).

Les extraits de safran connus dans l'art antérieur sont pour la plupart standardisés à 2% de safranal par spectrométrie UV selon la norme ISO 3632-2 :2010 qui surévalue la teneur en safranal de 20 à 50 fois (Garcia-Rodriguez et al., 2017 « Comparative evaluation of an ISO 3632 method and an HPLC-DAD method for safranal quantity determination in saffron »). C'est ainsi que de nouveaux extraits dont la teneur est mesurée par HPLC sont récemment apparus sur le marché, tels qu'un extrait commercialisé par la société Pharmactive titré à 0,03% (tel que décrit dans WO 2017/182688), mais encore par la société Activ'Inside titré à au moins 0,2% (tel que décrit dans FR 3054443). Lesdits extraits sont sur des supports maltodextrine ou gomme arabique.

Toutefois, l'efficacité de ces produits peut encore être améliorée pour permettre de lutter voire prévenir certains troubles tels que la mauvaise humeur, la dépression, l'anxiété, le stress, ou les troubles cognitifs. C'est l'objectif de l'invention.

**Résumé de l'invention**

Pour y répondre, l'invention propose de combiner un extrait de safran comprenant du safranal avec du cannabidiol.

Des études menées chez l'homme ont mis en évidence l'efficacité du cannabidiol (CBD), administré seul, sans Tétrahydrocannabinol (THC), à la dose de 300mg, dans la diminution des symptômes d'anxiété chez des adultes sains mis en situation de stress. A l'inverse, des doses plus faible (100mg) ou plus forte (900mg) ont été sans effet (Zuardi 2017).

Toutefois, il est connu que le corps humain possède des récepteurs très sensibles aux cannabinoïdes, en particulier les récepteurs CB1 et CB2 qui constituent avec leurs ligands endogènes, le « système endocannabinoïde ». Or, il n'est pas possible de prédire avec une certitude satisfaisante les effets d'une diminution ou d'une augmentation de l'activité du système endocannabinoïde dans une pathologie donnée.

En effet, pour une pathologie donnée, il a été mis en évidence soit un effet néfaste, soit un effet positif de l'activation des récepteurs dans les tissus et organes concernés par la pathologie. De plus, il a été observé que les niveaux de deux endocannabinoïdes endogènes de CB1 et CB2 (2-AG et anandamide) peuvent varier dans la même direction ou d'une façon totalement opposée (Di Marzo, 2008).

Par ailleurs, il est suggéré que la crocine, molécule présente dans le safran, pourrait agir comme agoniste des récepteurs CB1 et CB2 (Vafaei, 2020) ; alors qu'une autre étude à montrer que le safran induisait une diminution de l'expression génique des récepteurs CB1 et CB2 (Maccarone, 2016).

Ainsi, d'après l'état actuel des connaissances de l'homme de l'art, il n'est pas possible de prédire les effets biologiques associés à une administration de cannabinoïde tel que le CBD seule ou en combinaison avec du safran.

Pourtant, les inventeurs en travaillant sur le safran et le chanvre individuellement, ont de façon surprenante observé qu'un mélange de molécules obtenu d'un extrait de *Crocus sativus* comprenant une fraction volatile riche en terpènes, préférentiellement au moins 0,03% de safranal, plus préférentiellement au moins 0,2% de safranal mesuré par HPLC et d'un extrait de *Cannabis sativa,* dans un ratio spécifique, permet de réduire très significativement la dose d'extrait de *Crocus sativus* et/ou la dose d'extrait de *Cannabis sativa* par rapport aux doses nécessaires lorsque l'extrait de *Crocus sativus* ou l'extrait de *Cannabis sativa* est administré seul, et ainsi de répondre à ce besoin.

Les inventeurs ont également observé que ledit mélange de molécules permet d'améliorer l'effet d'entourage et ainsi d'améliorer la stabilité et la biodisponibilité des molécules présentes dans les extraits de *Crocus sativus* et de *Cannabis sativa,* et par conséquent une meilleure efficacité de doses inférieures aux doses efficaces individuellement.

Ainsi, l'invention concerne une composition comprenant un mélange de molécules comprenant au moins du safranal et au moins du cannabidiol, obtenu à partir d'extrait *Crocus sativus* et d'extrait de *Cannabis sativa* ou d'extrait de *Crocus sativus* et de *Cannabis sativa,* et présentant un ratio safranal/cannabidiol compris entre 0,03/1000 et 0,03, ledit extrait de *Cannabis sativa* comprenant au plus 0,2% de Tétrahydrocannabinol (THC) en poids de matière sèche par rapport au poids total de la matière sèche de l'extrait de *Cannabis sativa.*

Dans le cadre de leurs travaux, les inventeurs ont ainsi identifié le ratio safranal/cannabidiol compris entre 0,03/1000 et 0,03, comme étant particulièrement d'intérêt pour améliorer l'effet d'entourage et réduire les doses de cannabidiol et/ou de safranal.

Préférentiellement, l'invention concerne une composition comprenant un mélange de molécules comprenant au moins :

- un extrait de *Crocus sativus* comprenant au moins 0,03% de safranal, en poids par rapport au poids total de la matière sèche de l'extrait de *Crocus sativus,* mesurée par HPLC, et un extrait de *Cannabis sativa* comprenant au moins un cannabidiol, ou
- un extrait obtenu de *Crocus sativus* et de *Cannabis sativa* comprenant au moins 0,03% de safranal, en poids par rapport au poids total de la matière sèche de l'extrait de *Crocus sativus,* mesurée par HPLC et au moins un cannabidiol.

Plus préférentiellement, la composition selon l'invention comprend deux principes actifs respectivement à base d'extrait de *Crocus sativus* et d'extrait de *Cannabis sativa,* dans laquelle :

i. le premier principe actif est un extrait de *Crocus sativus* comprenant au moins 0,03% de safranal, en poids par rapport au poids total de la matière sèche de l'extrait de *Crocus sativus,* mesurée par HPLC, et
ii. le deuxième principe actif est un extrait de *Cannabis sativa* comprenant au moins un cannabidiol (CBD).

Encore plus préférentiellement, l'invention se rapporte à une composition constituée de deux principes actifs (c'est-à-dire sans autre principe actif) et d'au moins un excipient acceptable, dans laquelle :

i. le premier principe actif est un extrait de *Crocus sativus* comprenant le safranal, préférentiellement au moins 0,03% de safranal, en poids par rapport au poids total de la matière sèche de l'extrait de *Crocus sativus,* mesurée par HPLC, et
ii. le deuxième principe actif est un extrait de *Cannabis sativa* comprenant au moins un cannabidiol (CBD).

Selon une variante, la composition selon l'invention comprend un principe actif à base d'extrait de *Crocus sativus* et de *Cannabis sativa* comprenant le safranal, préférentiellement au moins 0,03% de safranal, en poids par rapport au poids total de la matière sèche de l'extrait de *Crocus sativus,* mesurée par HPLC, et le cannabidiol (CBD).

Le cannabidiol est l'un des nombreux cannabinoïdes retrouvés dans le chanvre (*Cannabis sativa*)*,* en particulier il s'agit du deuxième cannabinoïde en termes de concentration, après le tétrahydrocannabinol (THC). Les produits au CBD se sont démocratisés dans le monde et récemment en France, puisque ceux-ci ne sont pas des psychotropes comme peuvent l'être les produits à base de THC.

Les extraits comprenant du CBD, en particulier les extraits de *Cannabis sativa* peuvent être différenciés selon leur composition, tels que les extraits de *Cannabis sativa* à spectre complet (ou Full Spectrum CBD), les extraits de *Cannabis sativa* à spectre large (ou CBD Board Spectrum) ou l'isolat de CBD.

Préférentiellement, la présente invention se rapporte à une composition comprenant au moins un extrait de de *Cannabis sativa* tel qu'un extrait de *Cannabis sativa* à spectre large, ou l'isolat de CBD.

Enfin, selon un autre aspect, la présente composition selon l'invention vise à améliorer l'humeur, et/ou les performances cognitives ;et/ou prévenir et/ou traiter le stress, et/ou l'anxiété, et/ou la dépression, et/ou les troubles du sommeil, et/ou la mémoire, et/ou la démence, et/ou le déclin cognitif, et/ou les troubles digestifs, et/ou les troubles articulaires, et/ou les troubles érectiles, et/ou les troubles de la vision, et/ou les troubles liées à la ménopause ou liés au syndrome prémenstruel chez l'être humain ou l'animal et peut être utilisée pour ces effets.

D'autres caractéristiques et avantages ressortiront de la description détaillée de l'invention, des figures et des exemples qui vont suivre.

**Brève description des figures**

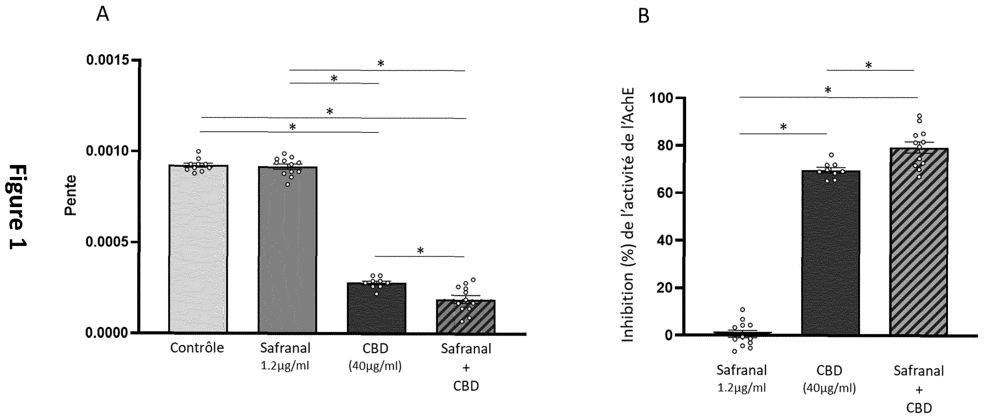

La figure 1 représente l'effet de la composition selon l'invention sur l'activité de l'AChE. Le panel A représente les pentes des courbes d'absorbances normalisées (moyenne ±SEM) dans leur partie linéaire, en fonction des différentes conditions testées. Le panel B représente les pourcentages d'inhibition (moyenne ±SEM) de l'activité de l'AchE par rapport au contrôle en fonction des différentes conditions testées. * p<0.05.

**Description détaillée de l'invention**

Définition

Par « isolat de CBD » au sens de l'invention, on entend un extrait de *Cannabis sativa* pur, c'est à dire un extrait issu de fleur, feuille, graine et tige de *Cannabis sativa* dans lequel le CBD est isolé de tous les autres cannabinoïdes. L'isolat comprend alors un taux très élevé de CBD d'au moins 90%, préférentiellement au moins 95%, plus préférentiellement au moins 98%, encore plus préférentiellement au moins 99%.

Par « extrait de *Cannabis sativa* à spectre large » ou « CBD à spectre large » ou « Broad Spectrum CBD » au sens de l'invention, on entend un extrait de toutes les parties (plante entière) de *Cannabis sativa* c'est-à-dire les fleurs, feuilles, graines et tiges, l'extrait comprenant d'autres cannabinoïdes en plus du CBD, tels que le cannabidol (CBN), le cannabigérol (CBG), le cannabichromène (CBC) à l'exclusion du Tétrahydrocannabinol (THC). Par « exclusion de Tétrahydrocannabinol », on entend au sens de l'invention une quantité de THC d'au plus la teneur maximale réglementaires autorisée, préférentiellement d'au plus 0,2% en poids de matière sèche par rapport au poids total de la matière sèche de l'extrait de *Cannabis sativa,* préférentiellement d'au plus 0,1%, plus préférentiellement d'au plus 0%. Cet extrait permet également de présenter l'effet d'entourage.

Par « cannabidol » ou « CBD » au sens de l'invention, on entend un cannabinoïde présent dans l'extrait de *Cannabis sativa,* en particulier un phytocannabinoïde bicyclique lipophile de formule 2-[(1R,6R)-6-isopropényl-3-méthylcyclohex-2-én-1-yl]-5-pentylbenzène-1,3-diol ; mais également son précurseur l'acide cannabidiolique, ou cannabidiol-acide (CBDA) ; mais également le CBC (cannabichromène), le CBG (cannabigerol) ou le CBN (cannabinol). Le CBDA est transformé en CBD par un processus de décarboxylation. Tous deux ne présentent pas d'effet psychoactif mais présentent un effet d'entourage.

Par « effet d'entourage » au sens de l'invention, on entend l'interaction particulière des différents terpènes, polyphénols et cannabinoïdes à l'exclusion du THC permettant de moduler l'ensemble des effets de *Cannabis sativa* sur l'organisme.

Par « poids total de la matière sèche de l'extrait » au sens de l'invention, la matière sèche est ce que l'on obtient lorsqu'on retire l'eau de la composition ou de l'extrait, elle peut être obtenue par perte à la dessication à 105°C. Le produit sec obtenu pour être sous forme solide ou liquide (dans le cas des extraits ou composition huileuse).

Par « excipient acceptable » au sens de l'invention on entend tout composé permettant de faciliter la mise en forme de la composition et ne modifiant pas la nature de l'activité biologique des deux principes actifs. Un excipient acceptable peut être un solvant, tampon, solution saline, plastifiant, lubrifiant, milieu de dispersion, agents retardant ou augmentant l'absorption, agent d'écoulement, agent isotonique, agent antioxydant, agent chélatant. Par exemple, il peut s'agir d'excipients pharmaceutiquement acceptables qui sont choisis selon la forme pharmaceutique et le mode d'administration souhaité, parmi les excipients habituels et connus de l'homme du métier et apte à un usage humain et/ou vétérinaire. L'excipient sera ainsi choisi en fonction de la voie d'administration, par exemple approprié pour une administration orale, intraveineuse, intramusculaire, topique etc. Enfin, il peut s'agir d'excipient acceptable dans les produits de nutrition, tels que les dextrines, la maltodextrine, le sucre, la silice, la glycérine, des matières grasses, des cires végétales, des huiles végétales hydrogénées ou non hydrogénées dont par exemple l'huile de chanvre, des protéines, des peptides, alginates, phospholipides, des polyols (par exemple sorbitol ou maltitol), l'amidon, la cellulose, le carbonate de calcium ou de magnésium, ou la gomme arabique.

Par « extrait de » au sens de l'invention, on entend au moins une molécule, préférentiellement un ensemble de molécules, obtenue(s) à partir de *Crocus sativus,* ou de *Cannabis sativa,* ou de *Crocus sativus* et de *Cannabis sativa.* La matière première peut-être les feuilles et/ou les fleurs et/ou les tiges et/ou les graines et/ou les stigmates et/ou les pétales, préférentiellement la matière première est les stigmates et/ou pétales et/ou fleurs de *Crocus sativus* et les fleurs, feuilles, graines et tiges de *Cannabis sativa.* Ainsi, dans le contexte de l'invention, la composition peut comprendre un mélange d'extrait de *Crocus sativus* et de *Cannabis sativa* ou un extrait issu d'un mélange de *Crocus sativus* et *Cannabis sativa.*

Par « ratio safranal/cannabidiol » au sens de l'invention, on entend le rapport de la quantité de safranal contenue dans la composition et la quantité de cannabidiol contenue dans la composition, en particulier le ratio est compris entre 0,03/1000 et 0,03. Par « ratio de 0,03 », au sens de l'invention, on entend un ratio de 0,03 plus ou moins 0,05.

Par « prévention » au sens de l'invention, on entend la réduction à un degré moindre du risque ou de la probabilité d'occurrence d'un phénomène donné, par exemple la mauvaise humeur, la dépression, le stress ou l'anxiété.

Par « traitement » au sens de l'invention, on entend une diminution de la progression de la maladie, une stabilisation, une inversion ou régression, voire une interruption ou inhibition de la progression de la dépression, de troubles du sommeil, de la démence, de troubles digestifs, de troubles articulaires, de troubles érectiles, de troubles de la vision ou de troubles liées à la ménopause ou liés au syndrome prémenstruel. Dans le contexte de l'invention, ces termes s'appliquent également sur un ou plusieurs symptômes desdites maladies de la présente invention.

Composition

La présente invention a donc pour objet une composition comprenant un mélange de molécules comprenant au moins :

- un extrait de *Crocus sativus* comprenant au moins du safranal, préférentiellement au moins 0,03% de safranal, en poids par rapport au poids total de la matière sèche de l'extrait de *Crocus sativus,* mesurée par HPLC, et un extrait de *Cannabis sativa* comprenant au moins un cannabidiol, ou
- un extrait obtenu de *Crocus sativus* et de *Cannabis sativa* comprenant au moins du safranal, préférentiellement au moins 0,03% de safranal, en poids par rapport au poids total de la matière sèche de l'extrait de *Crocus sativus,* mesurée par HPLC et au moins un cannabidiol,

ledit extrait de *Cannabis sativa* comprenant au plus 0,2% de Tétrahydrocannabinol (THC) en poids de matière sèche par rapport au poids total de la matière sèche de l'extrait de *Cannabis sativa,* ladite composition présentant un ratio safranal/cannabidiol compris entre 0,03/1000 et 0,03.

En particulier, la composition comprend deux principes actifs, dans laquelle :

a. le premier principe actif est un extrait de *Crocus sativus* comprenant au moins du safranal, préférentiellement au moins 0,03% de safranal, en poids par rapport au poids total de la matière sèche de l'extrait de *Crocus sativus,* mesurée par HPLC, et
b. le deuxième principe actif est un extrait de *Cannabis sativa* comprenant au moins un cannabidiol (CBD).

Selon une variante préférée, la composition est constituée de deux principes actifs (c'est-à-dire sans autre principe actif) et au moins un excipient acceptable, dans laquelle :

a. le premier principe actif est un extrait de *Crocus sativus* comprenant au moins du safranal, préférentiellement au moins 0,03% de safranal, en poids par rapport au poids total de la matière sèche de l'extrait de *Crocus sativus,* mesurée par HPLC, et
b. le deuxième principe actif est un extrait de *Cannabis sativa* comprenant au moins un cannabidiol (CBD).

L'extrait de *Crocus sativus* est obtenu préférentiellement à partir des stigmates et/ou pétales et/ou fleurs de *Crocus sativus* comprenant du safranal, en particulier au moins 0,03%, préférentiellement au moins 0,05%, plus préférentiellement au moins 0,1%, encore plus préférentiellement au moins 0,2% de safranal en poids par rapport au poids total de la matière sèche, ladite concentration étant mesurée par méthode HPLC.

L'extrait de *Crocus sativus* peut également comprendre, préférentiellement, d'autres molécules telles que :

- au moins 2% de crocines en poids par rapport au poids total de la matière sèche de l'extrait de *Crocus sativus,* mesurée par HPLC et pouvant être considéré comme la somme de la trans-crocin-4 (crocine majoritaire), trans-crocin-3, trans-crocin-2', cis-crocin-4, trans crocin-2, and trans-crocin-1, et/ou

- des flavonoïdes dérivés du kaempferol et/ou des dérivés de la picrocrocine et/ou des terpènes à l'exclusion du safranal.

Les flavonoïdes dérivés du kaempferol représentent préférentiellement au moins 500 ppm en poids de matière sèche de l'extrait, mesuré par méthode HPLC.

Les dérivés de la picrocrocine représentent préférentiellement au moins 0,5% en poids de matière sèche de l'extrait, mesurée par méthode HPLC.

Les terpènes à l'exclusion du safranal, représentent préférentiellement au moins 0,03% en poids de matière sèche de l'extrait, mesurée par méthode HPLC.

Par « extrait de safran » on entend au sens de l'invention un extrait obtenu à partir des stigmates et/ou pétales et/ou fleurs de *Crocus sativus,* en particulier au moins une molécule ou un ensemble de plusieurs molécules issue(s) des stigmates et/ou pétales et/ou fleurs comprenant du safranal. Il peut s'agir d'une sélection spécifique de molécules natives présentes dans la plante ou de molécules obtenues par tout type de transformation desdites molécules natives. La matière première utilisée pour obtenir l'extrait est les stigmates et/ou pétales et/ou fleurs de *Crocus sativus.*

Préférentiellement, l'extrait de *Crocus sativus* de la composition selon l'invention est encapsulé soit dans une phase aqueuse soit dans une phase grasse (par exemple huile végétale).

Selon un mode de réalisation préféré l'extrait de *Crocus sativus* a également subit un traitement thermique après son encapsulation d'au moins 2 heures à une température comprise entre 30°C et 95°C.

Lorsque l'extrait de *Crocus sativus* est uniquement encapsulé, l'agent pour encapsuler l'extrait est choisi parmi la gomme arabique, les cyclodextrines ou des matières grasses, les cires végétales, les huiles végétales hydrogénées ou non hydrogénées, l'huile, préférentiellement l'huile de chanvre, les protéines, les peptides, les dextrines, alginates, phospholipides.

Selon une variante, l'extrait de *Crocus sativus* a été obtenu par un procédé qui comprend une étape qui consiste à simultanément imprégner et encapsuler l'extrait de *Crocus sativus* dans la solution d'extraction et dans un support choisi parmi des dextrines, de la maltodextrine, des sucres, de la silice, de la gomme arabique, glycérine, des matières grasses, des cires végétales, des huiles végétales hydrogénées ou non hydrogénées dont par exemple l'huile de chanvre, des protéines, des peptides, dextrines, alginates, phospholipides plus préférentiellement la maltodextrine. Ledit extrait a également préférentiellement subit un traitement thermique après l'imprégnation et encapsulation d'au moins 2 heures à une température comprise entre 30°C et 95°C.

Lorsque l'extrait est imprégné sur un support, les pourcentages de molécules présentes dans l'extrait sont donnés en poids de matière sèche de l'extrait incluant le support.

Plus préférentiellement, l'extrait de *Crocus sativus* est un extrait obtenu selon le procédé décrit dans le brevet FR 3054443, incorporé par référence.

Ainsi, l'extrait est obtenu par la mise en œuvre des étapes suivantes réalisée à partir de matière première de *Crocus sativus* :

- Eventuellement séchage,
- Broyage, préférentiellement entre 50 et 500 μm,
- Extraction aqueuse ou hydroalcoolique ou avec un solvant organique ou avec une phase grasse de préférence de l'huile végétale,
- Imprégnation et/ou encapsulation de l'extrait obtenu dans la solution d'extraction sur un support,
- Traitement thermique, préférentiellement d'au moins 2 heures à une température comprise entre 30°C et 95°C.

L'étape de traitement thermique peut éventuellement être réalisée à tout moment de ce procédé tel que :

- Entre l'étape de séchage et de broyage, ou
- Entre l'étape de broyage et d'extraction, ou
- Entre l'extraction et l'étape d'imprégnation et encapsulation, ou
- Préférentiellement après l'étape d'imprégnation et encapsulation.

Selon un mode de réalisation particulièrement adapté, l'étape de traitement thermique dans la mise en œuvre de ce procédé est une étape de traitement thermique dans une étuve ou une cuve pendant au moins 2 heures, encore plus préférentiellement pendant au moins 24 heures à une température comprise entre 30°C et 95°C, encore plus préférentiellement à une température comprise entre 30°C et 60°C.

Le broyage peut être réalisé par tout moyen adapté connu, notamment par un broyeur à couteau, à broches ou à marteau, préférentiellement un broyeur à broches.

L'étape d'extraction peut être réalisée par tout moyen adapté connu. Dans le cas d'une extraction aqueuse, la matière broyée est introduite dans l'eau à raison de 50g/L.

Dans le cas d'une extraction hydroalcoolique, le solvant peut être notamment de l'éthanol, préférentiellement de l'éthanol à 60% v/v. La matière broyée est introduite dans la solution hydroalcoolique à raison de 50g/L.

Dans le cas d'une extraction avec un solvant organique, le solvant peut être notamment du méthanol ou de l'acétate d'éthyle, préférentiellement du méthanol à 30% v/v. La matière broyée est introduite dans le solvant organique à raison de 100g/L.

Après extraction, le procédé peut également comprendre une étape d'acidification. Cette étape consiste en l'ajout d'acide dans le solvant aqueux ou hydroalcoolique. Elle permet de diminuer le pH de la solution d'extraction entre 3 et 5. Elle peut être notamment réalisée dans les conditions suivantes : ajout d'acide citrique ou acide chlorhydrique dans le solvant hydroalcoolique pour ajuster le pH à 4.

Dans le cas d'une extraction avec une phase grasse, la phase grasse peut être notamment de l'huile d'olive, de l'huile de chanvre, de l'huile de coco, de l'huile de tournesol de l'huile de colza. La solution peut être chauffée à au moins 60°C. Selon un mode de réalisation préféré, une telle extraction peut permettre d'obtenir un extrait de *Crocus sativus* et de *Cannabis sativa.*

L'étape d'imprégnation et d'encapsulation simultanément sur le support consiste en l'ajout d'un agent de charge dans la solution d'extraction, c'est-à-dire à l'état liquide. Plus préférentiellement une telle étape est également mise en œuvre simultanément avec l'étape d'extraction ce qui permet de piéger les molécules de safranal d'une part et de crocines d'autre part lors de l'extraction. Le piégeage des molécules de safranal ayant pour conséquence d'obtenir un extrait fortement concentré en safranal d'au moins 0,2% mesuré par HPLC et encapsulé permettant une meilleure stabilité à la fois du safranal et des crocines.

Le support ou agent de charge ou excipient peut être notamment choisi parmi les constituants suivants : maltodextrine, sucre, silice, gomme arabique, des cyclodextrines ou des matières grasses, cires végétales, huiles végétales hydrogénées ou non hydrogénées dont par exemple l'huile de chanvre, des protéines, des peptides, dextrines, alginates, phospholipides préférentiellement la maltodextrine. Des antioxydants et/ou des agents chélatants peuvent être ajoutés pour permettre une meilleure stabilité des molécules d'intérêt. Cette étape consiste en l'agitation à haute vitesse de la solution d'extraction contenant l'agent de charge de l'excipient ou du support.

Une double émulsion peut également être envisagée avec des agents tensio-actifs connus de l'homme de l'art pour permettre une solubilisation dans de l'eau de l'extrait initialement encapsulé ou microencapsulé constituant ainsi une double encapsulation ou microencapsulation. La microencapsulation permet de protéger les substances actives au sein de particules de tailles comprises entre $1\mu m$ et $1000\ \mu m$, préférentiellement entre $1\ \mu m$ et $500\mu m$ et plus préférentiellement entre $5\mu m$ et $100\mu m$, encore plus préférentiellement entre $5\mu m$ et $50\mu m$.

Les molécules issues du deuxième principe actif présent dans la composition selon l'invention est obtenu à partir de chanvre, en particulier de *Cannabis sativa.* Celui-ci contient différents métabolites d'intérêt, qui peuvent être classés en trois grandes familles : les cannabinoïdes (dits « phytocannabinoïdes »), les terpènes et les polyphénols.

Les cannabinoïdes de *Cannabis sativa* incluent notamment le THC et le CBD et vont agir sur les récepteurs CB1 et CB2. CB1 est majoritairement localisé au niveau du système nerveux central et des terminaisons nerveuses périphériques, alors que CB2 est essentiellement retrouvé dans les cellules du système immunitaire et la rate.

Le THC est le composant à l'origine des effets psychotropes du cannabis, conférant des effets d'euphorie et de relaxation. Cependant, la consommation de THC induit aussi une altération des fonctions cognitives. A la différence du THC, le CBD ne possède pas d'effets psychotropes malgré des effets bien-être tels que la réduction du stress, la détente, ou encore l'amélioration du sommeil. Il aide également à supporter certaines douleurs chroniques. Par ailleurs, le CBD, consommé avant la consommation de THC, serait capable d'atténuer les effets euphorisants du THC mais également de contre-carrer ses effets délétères tels que l'altération des performances psychomotrices, l'anxiété et la psychose.

Préférentiellement, l'extrait de *Cannabis sativa* selon l'invention est un extrait de *Cannabis sativa* à spectre large ou un isolat de CBD.

Ainsi, il ne comprend pas de THC, c'est-à-dire que la quantité de THC présente dans ledit extrait est d'au plus la teneur maximale réglementaires autorisée, préférentiellement d'au plus 0,2%, de façon la plus préférée d'au plus 0%.

Préférentiellement, l'extrait de *Cannabis sativa* selon l'invention comprend au moins 90% de cannabidiol ou entre 0,1% et 50% de cannabidiol, plus préférentiellement entre 0,1% et 10% de cannabidiol exprimé en en poids par rapport au poids total de la matière sèche de l'extrait de *Cannabis sativa.*

L'extrait de *Cannabis sativa* comprend également des terpènes.

Parmi les terpènes présents dans la majorité des variétés de *Cannabis sativa,* on peut citer le monoterpène myrcène (ci-après dénommé myrcène) ainsi que les sesquiterpènes β-caryophyllène et α-humulène. Les monoterpènes α-pinène, le limonène et le linalool, de même que le sesquiterpène bisabolol, sont également des terpènes communs. Ledit extrait de *Cannabis sativa* ne comprend pas de safranal.

Préférentiellement, l'extrait de *Cannabis sativa* comprend au moins 0,001% de terpènes, en poids par rapport au poids total de la matière sèche de l'extrait de *Cannabis sativa,* plus préférentiellement au moins 0,01% de terpènes, encore plus préférentiellement au moins 0,05% de terpènes.

Selon un mode particulièrement préféré, il comprend entre 0,1% et 10% de cannabidiol et au moins 0,001% de terpènes en poids par rapport au poids total de la matière sèche de l'extrait de *Cannabis sativa,* plus préférentiellement au moins 0,01% de terpènes, encore plus préférentiellement au moins 0,05% de terpènes.

Parmi la multitude de terpènes présents dans *Cannabis sativa,* l'extrait de *Cannabis sativa* présent dans la composition selon l'invention, comprend le myrcène, celui-ci représente préférentiellement au moins 5ppm, plus préférentiellement au moins 100 ppm, en poids par rapport au poids total de la matière sèche de l'extrait de *Cannabis sativa.*

En outre, les terpènes majoritairement présents dans l'espèce *Cannabis sativa* ne sont pas retrouvés dans l'espèce *Crocus sativus,* en particulier le myrcène n'est pas présent dans l'extrait de *Crocus sativus,* présent dans la composition selon l'invention.

Les derniers métabolites d'intérêt présents dans *Cannabis sativa* sont les polyphénols. Le chanvre comporte une vingtaine de flavonoïdes différents, dont certains lui sont spécifiques comme les cannflavines A et B (connus pour leurs propriétés anti-inflammatoires). On peut également retrouver des quercétines, du kaempférol, des catéchines, ou encore de la lutéoline connus pour leurs propriétés nootropiques. Les polyphénols présents dans *Cannabis sativa* contribuent également, avec les cannabinoïdes et les terpènes, à l'effet d'entourage.

Pour obtenir la composition selon l'invention, l'extrait de *Crocus sativus* est mélangé avec l'extrait de *Cannabis sativa* et éventuellement au moins un excipient ou l'extrait est issu d'un mélange de *Crocus sativus* et de *Cannabis sativa* et éventuellement au moins un excipient.

Le ratio safranal/cannabidiol dans la composition est compris entre 0,03/100 et 0,03, plus préférentiellement celui-ci est compris entre 0,03/25 et 0,03. Un tel ratio permet d'améliorer encore plus les effets synergiques observés entre l'extrait de *Crocus sativus* comprenant au moins 0,2% de safranal, mesuré par HPLC et l'extrait de *Cannabis sativa.* En particulier, il permet d'obtenir un effet d'entourage amélioré ce qui, permet de réduire très significativement la dose de CBD et/ ou la dose d'extrait de *Crocus sativus,* en particulier de safranal, par rapport aux doses nécessaires lorsque le CBD ou l'extrait de *Crocus sativus* est administré seul.

Plus préférentiellement, la composition selon l'invention présente un ratio safranal/myrcène supérieur à 0,03 préférentiellement supérieur à 0,1, plus préférentiellement supérieur à 1.

La composition selon l'invention peut être préparée par la mise en œuvre des étapes suivantes :

    a. préparation de l'extrait de *Crocus sativus* tel que décrit précédemment,
    b. préparation de l'extrait de *Cannabis sativa* tel que décrit précédemment,
    c. mélange des deux extraits,
    d. ajout d'un agent antioxydant
    e. éventuellement, une étape d'encapsulation des deux principes actifs et de l'agent antioxydant.

Selon une variante, la composition selon l'invention peut être préparée par la mise en œuvre des étapes suivantes :

    a. préparation de l'extrait de *Crocus sativus* et de *Cannabis sativa* de façon simultanée,
    b. ajout d'un agent antioxydant,
    c. éventuellement, une étape d'encapsulation dudit extrait et de l'antioxydant.

La composition selon l'invention peut également comprendre un agent antioxydant (ou antioxydant) et/ou chélatant choisi parmi les substances suivantes : acide ascorbique E300, $\alpha$-tocophérol (vitamine E E306), E309, extrait de romarin E392, extrait de crocus sativus (et plus particulièrement de pétales) contenant des flavonols, et plus particulièrement des kaempférols, BHA E320, BHT E321, E330 et de l'acide citrique ou l'EDTA ou des protéines en tant qu'agent chélateurs. Les protéines sont préférentiellement choisies parmi des extraits protéiques de son de riz, de germe de blé hydrolysé, de son d'orge, de son d'avoine, hydrolysat de protéine de soja tel que décrit par Mallory E Walters et al. 2018 « Potential of Food Hydrolyzed Proteins and Peptides to Chelate Iron or Calcium and Enhance their Absorption ». L'agent antioxydant et/ou le chélatant permet ainsi de stabiliser et conserver dans le temps le safranal, les crocines, le CBD, les terpènes et les polyphénols présents dans la composition afin de maintenir dans le temps les effets biologiques associés, y compris les effets synergiques en combinaison.

Préférentiellement, l'antioxydant est présent dans la composition selon l'invention de façon à ce que le ratio d'antioxydant sur safranal soit de 1/100 à 10/1, en poids de matière sèche de safranal, mesuré par analyse HPLC.

L'antioxydant et/ou le chélatant représente préférentiellement 0,01g/100g de crocines à 25g/100g en poids de matière sèche de crocines, mesuré par méthode HPLC.

A titre d'exemple, la vitamine antioxydante peut être la vitamine C et/ou la vitamine E.

A titre d'exemple, les polyphénols issus de l'extrait de *Crocus sativus* et/ou de *Cannabis sativa* peuvent être des flavonoïdes tels que les cannflavines A et B, la quercétine, le kaempférol, les catéchines et/ou la lutéonine.

La composition selon l'invention peut se présenter sous une forme sèche, liquide ou gel.

Préférentiellement, la composition selon l'invention se présente sous forme de liquide, de liquide huileux, de liquide pour cigarette électronique, de poudre, de capsule molle, de gélule, de comprimé, de stick, de sachet, de « gummies » (gommes à mâcher), de crème, de lotion, d'huile, de gel, de patch transdermique, de plat préparé, de boisson.

La composition selon l'invention peut éventuellement comprendre d'autres composants adaptés connus, tels que des excipients, sélectionnés en fonction de la forme et de l'utilisation envisagée de la composition.

Selon un objet préféré, la composition comprend au moins 15mg d'extrait de *Crocus sativus* et au moins 3 mg de CBD. Préférentiellement, ladite composition est administrée par jour ou par prise à un être humain.

Ladite composition peut être administrée par exemple en une ou deux prises.

La composition peut être utilisé dans de nombreuses applications. Ainsi l'invention a pour objet une composition selon l'invention pour son utilisation dans le traitement ou la prévention d'au moins un trouble choisi parmi la dépression, l'anxiété, le stress, les troubles de l'humeur (y compris les troubles pathologiques de l'humeur), les troubles de la mémoire, les troubles érectiles, les troubles liées à la ménopause ou liés au syndrome prémenstruel, les troubles du sommeil, les troubles digestifs, les troubles articulaires, les troubles de la vision, la démence, le déclin cognitif (y compris les troubles pathologiques du déclin cognitif), et leurs combinaisons chez l'être humain ou l'animal.

La composition peut également améliorer l'humeur non pathologique, et/ou les performances cognitives, et/ou la mémoire et/ou le déclin cognitif non pathologique lié à l'âge chez l'être humain ou l'animal.

L'invention est à présent illustrée par des exemples non limitatifs de compositions selon l'invention.

**Exemples**

Exemple 1 - Composition selon l'invention

Un premier exemple de composition selon l'invention est obtenu par la mise en œuvre du procédé consistant en la mise en œuvre des étapes suivantes :

    a. Obtention d'un extrait de *Crocus sativus* selon le procédé décrit dans le brevet FR 3054443

    b. Obtention d'un extrait de *Cannabis sativa* par la mise en œuvre des étapes suivantes :

    • un procédé d'extraction par solvants organiques tel que l'éthanol ou par un mélange hydroalcoolique tel que décrit dans Glivar et al., 2020 ou par l'intermédiaire de $CO_2$ supercritique tel que décrit par Nogueira et al., 2018 ou encore, de par sa nature lipophile, par une huile de préférence végétale telle que l'huile d'olive tel que décrit dans Casiraghi et al., 2018.

    • la décarboxylation du CBDA par l'application d'un procédé thermique durant ou après extraction, par des température de 100 à 140°C (le procédé thermique visant à décarboxyler le CBDA peut également être appliqué sur la matière première, avant extraction). L'extraction à l'huile peut aussi faire intervenir cette décarboxylation par un chauffage de l'huile à une température au moins égale à 110°C pendant un minimum de 40min.

    c. Mélange des extraits de *Crocus sativus* et de *Cannabis sativa* obtenus respectivement lors des étapes a et b.

La composition comprend alors les caractéristiques suivantes, pour une dose journalière :

- 15 mg d'extrait de *Crocus sativus* titré à 0,2% de safranal mesuré par HPLC, soit l'équivalent de 0,03mg de safranal,
- 10 mg d'un extrait de *Cannabis sativa* titré à 10% de CBD et 0,2% de myrcène, soit l'équivalent de 1mg de CBD et 0,02 mg de myrcène.

Exemple 2 - Composition selon l'invention

Un deuxième exemple de composition selon l'invention est obtenu par la mise en œuvre du procédé consistant en la mise en œuvre des étapes suivantes :

    a. extraction et chauffage dans un corps gras de façon simultanée, de *Crocus sativus* et de *Cannabis sativa,* à une température minimale de 80°C,

    b. obtention d'un extrait du mélange de *Crocus sativus* et de *Cannabis sativa.*

La composition comprend alors les caractéristiques suivantes :

- 10 mg de safran titré à 0,03% de safranal mesuré par HPLC, soit l'équivalent de 0,003mg de safranal
- 26,3 mg d'un isolat de *Cannabis sativa* titré à 95% de CBD et 0,038% de myrcène, soit l'équivalent de 25mg de CBD et 0,01 mg de myrcène
- 0,03μg d'extrait de romarin.

Exemple 3 - Composition selon l'invention

Un troisième exemple de composition selon l'invention est obtenu par la mise en œuvre de l'un des procédés décrits dans les exemples 1 et 2.

La composition comprend alors les caractéristiques suivantes :

- 10 mg d'un extrait de *Crocus sativus* titré à 0,2% de safranal mesuré par HPLC, soit l'équivalent de 0,02mg de safranal
- 26,3 mg d'un isolat de *Cannabis sativa* titré à 95% de CBD et 0,038% de myrcène, soit l'équivalent de 25mg de CBD et 0,01 mg de myrcène
- 0,3μg d'extrait de romarin

Exemple 4 - Composition selon l'invention

Un quatrième exemple de composition selon l'invention est obtenu par la mise en œuvre de l'un des procédés décrits dans les exemples 1 et 2.
La composition comprend alors les caractéristiques suivantes :

- 10 mg d'un extrait de *Crocus sativus* titré à 0,2% de safranal mesuré par HPLC, soit l'équivalent de 0,02mg de safranal
- 150 mg d'un isolat de *Cannabis sativa* titré à 10% de CBD et 0,32% de myrcène, soit l'équivalent de 15mg de CBD et 0,48 mg de myrcène
- 15μg de vitamine E (α-tocophérol)

Exemple 5 - Composition selon l'invention

Un cinquième exemple de composition selon l'invention est obtenu par la mise en œuvre de l'un des procédés décrits dans les exemples 1 et 2.
La composition comprend alors les caractéristiques suivantes :

- 10 mg d'un extrait de *Crocus sativus* titré à 0,2% de safranal mesuré par HPLC, soit l'équivalent de 0,02mg de safranal
- 20,2 mg d'un isolat de *Cannabis sativa* titré à 99% de CBD, soit l'équivalent de 20mg de CBD
- 3μg de vitamine E (α-tocophérol)

Exemple 6 - Composition nutritionnelle selon l'invention

Un sixième exemple de composition selon l'invention est obtenu par l'incorporation de la composition selon l'exemple 1 dans une recette de cookies au chocolat, consommés par l'Homme à raison de 2 cookies par jour.
Ladite recette consiste, pour 10 cookies, en :

- 200g de farine
- 10g de levure chimique
- 40g de poudre de coco
- 125g de pépites de chocolat (noir ou lait, selon vos goûts)
- 80g de sucre
- 1 œuf
- 120g de beurre

Exemple 7 - Composition de liquide pour cigarette électronique selon l'invention

Un septième exemple de composition selon l'invention est obtenu par l'incorporation de la composition selon l'exemple 1 dans une formule de liquide pour cigarette électronique destiné à l'Homme à raison de 2 ml de liquide par jour.
Ladite formule de liquide pour cigarette électronique consiste, pour 1 flacon de 10 ml, en :

- 8 ml de propylène glycol,
- 1 ml de glycérine végétal
- 0,5 g d'arômes alimentaires
- 100 mg de nicotine
- Ethanol et/ou eau déminéralisée : qsp 10 ml.

Exemple 8 - Composition de liquide pour cigarette électronique selon l'invention

Un huitième exemple de composition selon l'invention est obtenu par l'incorporation de la composition selon l'exemple 1 dans une formule de liquide pour cigarette électronique destiné à l'Homme à raison de 2 ml de liquide par jour.

Ladite formule de liquide pour cigarette électronique consiste, pour 1 flacon de 10 ml, en :

- 8 ml de propylène glycol,
- 1 ml de glycérine végétal
- 0,5 g d'arômes alimentaires

Ethanol et/ ou eau déminéralisée : qsp 10 ml.

## EVALUATION DE L'EFFICACITE DE LA COMPOSITION SELON L'INVENTION

Essai 1 : Evaluation de l'effet de la composition selon l'invention sur l'activité de l'acétylcholinestérase.

L'acétylcholine est un neurotransmetteur qui joue un rôle majeur dans la mémoire et la mémorisation. Dans les processus neurodégénératifs tels que les démences, les taux d'acétylcholine sont diminués expliquant ainsi l'apparition des symptômes cognitifs et psycho-comportementaux. L'un des objectifs des traitements actuels est de maintenir une neurotransmission acétylcholinergique normal en inhibant sa dégradation, soit en inhibant l'activité de l'acétylcholinestérase (enzyme responsable de la dégradation de l'acétylcholine).

L'objectif de cet essai est de comparer l'activité de l'acétylcholinestérase (AchE) en présence de la composition selon l'invention, présentant un ratio safranal/cannabidiol (CBD) de 0.03, avec l'activité de l'acétylcholinestérase en présence de safranal ou de CBD seul.

L'activité de l'AchE a été suivie et mesurée *in vitro* par une réaction colorimétrique suivant la méthode de Ellman. L'acétylthiocholine (ATCl) est dégradée en thiocholine et en acide acétique par l'AChE. La thiocholine réagit ensuite avec l'ion dithiobisnitrobenzoïque (DTNB) et l'ion produit (TNB) donne une coloration jaune. Dans une plaque 96 puits, les quantités suivantes ont été déposées dans chaque puits : $55\mu$l tampon (Tris-HCl 50mM), $75\mu$l DTNB (5mM), $25\mu$l AchE ($0,22$U/ml), $25\mu$l d'échantillon à tester (tampon ou safranal ($1.2\mu$g/ml), ou CBD ($40\mu$g/ml), ou safranal ($1.2\mu$g/ml) + CBD ($40\mu$g/ml).

Au dernier moment, $25\mu$L d'ATCl (1mM) a été ajouté pour initier la réaction. Par la suite, un suivi de l'absorbance à 405nM a été réalisé pendant 20min. Les résultats ont été analysés de la manière suivante. Pour chaque puits, les valeurs d'absorbance au cours du temps ($A_x$) sont normalisées par rapport à la première mesure d'absorbance ($A_{T0}$) selon la formule suivante : Absorbance normalisée ($A_n$) = $A_x - A_{T0}$. Le pourcentage d'inhibition de l'AchE a été déterminé en comparant les pentes des courbes (dans la partie linéaire) par rapport au contrôle selon l'équation suivante [Math 1].

$$[Math\ 1]$$

$$\%\text{inhibition} = \frac{Pente_{(Contrôle)} - Pente_{(échantillon)}}{Pente_{(échantillon)}} \times 100$$

Les valeurs des pentes et des pourcentages d'inhibition ont été comparées statistiquement en utilisant des analyses de variance (ANOVA). Une valeur de $p < 0.05$ est considérée comme significative.

Les résultats montrent qu'en présence de safranal l'activité de l'AchE n'est pas modifiée par rapport au contrôle (Figure 1). En revanche, en présence de CBD une diminution significative (69.8%) de l'activité de l'AchE a été observée. Enfin, en présence de la composition selon l'invention, l'inhibition de l'activité de l'acétylcholinestérase est significativement plus importante qu'en présence du CBD seul (79.4% vs 69.8%). De tels résultats démontrent la présence d'interactions synergiques entre le safranal et le CBD sur l'activité de l'AchE en permettant de diminuer plus significativement son activité. La baisse de son activité par la composition selon l'invention permet ainsi d'améliorer la transmission cholinergique et par conséquent les fonctions cognitives.

## Revendications

1. Composition comprenant un mélange de molécule comprenant au moins :

   - un extrait de *Crocus sativus* comprenant au moins du safranal, et un extrait de *Cannabis sativa* comprenant au moins un cannabidiol, ou
   - un extrait obtenu de *Crocus sativus* et de *Cannabis sativa* comprenant au moins du safranal, et au moins un cannabidiol,
   ledit extrait de *Cannabis sativa* comprenant au plus 0,2% de Tétrahydrocannabinol (THC) en poids de matière sèche par rapport au poids total de la matière sèche de l'extrait de *Cannabis sativa,*
ladite composition comprenant un ratio safranal/cannabidiol compris entre 0,03/1000 et 0,03.

2. Composition selon la revendication précédente **caractérisé en ce que** l'extrait de *Crocus sativus* ou l'extrait obtenu de *Crocus sativus* et de *Cannabis sativa* comprend au moins 0,03% de safranal, en poids par rapport au poids total de la matière sèche de l'extrait de *Crocus sativus,* mesurée par HPLC.

3. Composition selon l'une des revendications précédentes, **caractérisée en ce que** l'extrait de *Cannabis sativa* est un extrait de *Cannabis sativa* à spectre large ou un isolat de CBD.

4. Composition selon l'une des revendications précédentes, **caractérisée en ce que** l'extrait de *Cannabis sativa* comprend :

   - entre 0,1% et 10% de cannabidiol, exprimé en poids par rapport au poids total de la matière sèche de l'extrait de *Cannabis sativa,* ou
   - au moins 90% de cannabidiol, exprimé en poids par rapport au poids total de la matière sèche de l'extrait de *Cannabis sativa.*

5. Composition selon la revendication précédente, **caractérisée en ce que** l'extrait de *Cannabis sativa* comprend entre 0,1% et 10% de cannabidiol et au moins 0,001% de terpènes, en poids par rapport au poids total de la matière sèche de l'extrait de *Cannabis sativa.*

6. Composition selon la revendication précédente, **caractérisée en ce que** les terpènes représentent au moins 0,01% en poids par rapport au poids total de la matière sèche de l'extrait de *Cannabis sativa.*

7. Composition selon la revendication précédente, **caractérisée en ce que** les terpènes comprennent au moins des myrcènes.

8. Composition selon l'une des revendications précédentes, **caractérisée en ce que** l'extrait de *Crocus sativus* comprend

   - au moins 2% de crocines en poids par rapport au poids total de la matière sèche de l'extrait de *Crocus sativus,* mesurée par HPLC, et/ou
   - des flavonoïdes dérivés du kaempferol et/ou des dérivés de la picrocrocine et/ou des terpènes à l'exclusion du safranal.

9. Composition selon l'une des revendications précédentes, **caractérisée en ce que** l'extrait de *Crocus sativus* est encapsulé avec un agent choisi parmi la gomme arabique, les cyclodextrines, des matières grasses, les cires végétales, les huiles végétales hydrogénées ou non hydrogénées, l'huile de chanvre, les protéines, les peptides, les dextrines, alginates, phospholipides.

10. Composition selon l'une des revendications précédentes, **caractérisée en ce que** la composition comprend également un agent antioxydant.

11. Composition selon l'une des revendications précédentes, **caractérisée en ce que** la composition comprend au moins 15mg d'extrait de *Crocus sativus* et au moins 3 mg de CBD.

12. Composition selon l'une des revendications précédentes, **caractérisée en ce que** la composition se présente sous une forme sèche, liquide ou gel.

13. Composition selon la revendication précédente, **caractérisée en ce qu'**elle se présente sous forme de liquide, de liquide huileux, de liquide pour cigarette électronique, de poudre, de capsule molle, de gélule, de comprimé, de stick, de sachet, de gommes à mâcher, de crème, de lotion, d'huile, de gel, de patch transdermique, de plat préparé ou de boisson.

14. Composition selon l'une des revendications précédentes pour son utilisation pour prévenir et/ou traiter le stress, et/ou l'anxiété, et/ou la dépression, et/ou les troubles du sommeil, et/ou la démence, et/ou le déclin cognitif, et/ou les troubles digestifs, et/ou les troubles articulaires, et/ou les troubles érectiles, et/ou les troubles de la vision, et/ou les troubles liées à la ménopause ou liés au syndrome prémenstruel chez l'être humain ou l'animal.

15. Utilisation non thérapeutique d'une composition selon l'une des revendications 1 à 13 pour améliorer l'humeur et/ou les performances cognitives.

**Patentansprüche**

1. Zusammensetzung, umfassend eine Mischung von Molekülen, mindestens umfassend:

    - einen Extrakt von *Crocus sativus,* mindestens umfassend Safranal, und einen Extrakt von *Cannabis sativa,* mindestens umfassend ein Cannabidiol, oder
    - einen Extrakt von *Crocus sativus* und *Cannabis sativa,* umfassend mindestens Safranal und mindestens ein Cannabidiol,

    wobei der Extrakt von *Cannabis sativa* höchstens 0,2 Gew.-% in Trockenmasse Tetrahydrocannabinol (THC), bezogen auf das Gesamtgewicht der Trockenmasse des Extrakts von *Cannabis sativa,* umfasst, wobei die Zusammensetzung ein Safranal/Cannabidiol-Verhältnis zwischen 0,03/1000 und 0,03 umfasst.

2. Zusammensetzung nach dem vorstehenden Anspruch,
   **dadurch gekennzeichnet, dass** der Extrakt von *Crocus sativus* oder der Extrakt von *Crocus sativus* und *Cannabis sativa* mindestens 0,03 Gew.-% Safranal umfasst, bezogen auf das Gesamtgewicht der Trockenmasse des Extrakts von *Crocus sativus,* gemessen mittels HPLC.

3. Zusammensetzung nach einem der vorstehenden Ansprüche,
   **dadurch gekennzeichnet, dass** der Extrakt von *Cannabis sativa* ein Breitband-Extrakt von *Cannabis sativa* oder ein CBD-Isolat ist.

4. Zusammensetzung nach einem der vorstehenden Ansprüche,
   **dadurch gekennzeichnet, dass** der Extrakt von *Cannabis sativa* umfasst:

    - zwischen 0,1 Gew.-% und 10 Gew.-% Cannabidiol, bezogen auf das Gesamtgewicht der Trockenmasse des Extrakts von *Cannabis sativa* ausgedrückt, oder
    - mindestens 90 Gew.-% Cannabidiol, bezogen auf das Gesamtgewicht der Trockenmasse des Extrakts von *Cannabis sativa* ausgedrückt.

5. Zusammensetzung nach dem vorstehenden Anspruch,
   **dadurch gekennzeichnet, dass** der Extrakt von *Cannabis sativa* zwischen 0,1 Gew.-% und 10 Gew.-% Cannabidiol und mindestens 0,001 Gew.-% Terpene, bezogen auf das Gesamtgewicht der Trockenmasse des Extrakts von *Cannabis sativa* umfasst.

6. Zusammensetzung nach dem vorstehenden Anspruch,
   **dadurch gekennzeichnet, dass** die Terpene mindestens 0,01 Gew.-%, bezogen auf das Gesamtgewicht der Trockenmasse des Extrakts von *Cannabis sativa,* ausmachen.

7. Zusammensetzung nach dem vorstehenden Anspruch,
   **dadurch gekennzeichnet, dass** die Terpene mindestens Myrcene umfassen.

8. Zusammensetzung nach einem der vorstehenden Ansprüche,
   **dadurch gekennzeichnet, dass** der Extrakt von *Crocus sativus* umfasst:

    - mindestens 2 Gew.-% Crocine, bezogen auf das Gesamtgewicht der Trockenmasse des Extrakts von *Crocus sativus,* gemessen mittels HPLC, und/oder
    - Flavonoide, die aus Kaempferol und/oder Derivaten von Picrocrocin und/oder Terpenen, ausgenommen Safranal, gewonnen werden.

9. Zusammensetzung nach einem der vorstehenden Ansprüche,
   **dadurch gekennzeichnet, dass** der Extrakt von *Crocus sativus* mit einem Wirkstoff verkapselt ist, der ausgewählt ist aus Gummi arabicum, Cyclodextrinen, Fetten, pflanzlichen Wachsen, hydrierten oder nicht hydrierten Pflanzenölen, Hanföl, Proteinen, Peptiden, Dextrinen, Alginaten und Phospholipiden.

10. Zusammensetzung nach einem der vorstehenden Ansprüche,
**dadurch gekennzeichnet, dass** die Zusammensetzung auch ein Antioxidationsmittel umfasst.

11. Zusammensetzung nach einem der vorstehenden Ansprüche,
**dadurch gekennzeichnet, dass** die Zusammensetzung mindestens 15 mg des Extrakts von *Crocus sativus* und mindestens 3 mg CBD umfasst.

12. Zusammensetzung nach einem der vorstehenden Ansprüche,
**dadurch gekennzeichnet, dass** die Zusammensetzung in trockener, flüssiger oder Gelform vorliegt.

13. Zusammensetzung nach dem vorstehenden Anspruch,
**dadurch gekennzeichnet, dass** sie als Flüssigkeit, ölige Flüssigkeit, E-Zigaretten-Flüssigkeit, Pulver, Weichkapsel, Kapsel, Tablette, Stick, Beutel, Kaugummi, Creme, Lotion, Öl, Gel, transdermales Pflaster, Fertiggericht oder Getränk angeboten wird.

14. Zusammensetzung nach einem der vorstehenden Ansprüche zur Verwendung bei der Vorbeugung und/oder Behandlung von Stress und/oder Angstzuständen und/oder Depression und/oder Schlafstörungen und/oder Demenz und/oder kognitivem Abbau und/oder Verdauungsstörungen und/oder Gelenkerkrankungen und/oder Erektionsstörungen und/oder Sehstörungen und/oder Beschwerden im Zusammenhang mit der Menopause oder dem prämenstruellen Syndrom bei Menschen oder Tieren.

15. Nicht-therapeutische Verwendung einer Zusammensetzung nach einem der Ansprüche 1 bis 13 zur Verbesserung der Stimmung und/oder der kognitiven Leistungsfähigkeit.


**Claims**

1. Composition comprising a mixture of molecules comprising at least:

   - an extract of *Crocus sativus* comprising at least safranal, and an extract of *Cannabis sativa* comprising at least one cannabidiol, or
   - an extract obtained from *Crocus sativus* and from *Cannabis sativa* comprising at least safranal and at least one cannabidiol,

   said *Cannabis sativa* extract comprising at most 0.2% Tetrahydrocannabinol (THC) by weight of dry matter relative to the total weight of dry matter of the *Cannabis sativa* extract,
   said composition comprising a safranal/cannabidiol ratio of between 0.03/1000 and 0.03.

2. Composition according to the preceding claim, **characterized in that** the *Crocus sativus* extract or the extract obtained from *Crocus sativus* and from *Cannabis sativa* comprises at least 0.03% safranal by weight relative to the total weight of dry matter of the *Crocus sativus* extract, measured by HPLC.

3. Composition according to one of the preceding claims, **characterized in that** the *Cannabis sativa* extract is a broad-spectrum *Cannabis sativa* extract or a CBD isolate.

4. Composition according to one of the preceding claims,
**characterized in that** the *Cannabis sativa* extract comprises:

   - between 0.1% and 10% cannabidiol, expressed by weight relative to the total weight of dry matter of the *Cannabis sativa* extract, or
   - at least 90% cannabidiol, expressed by weight relative to the total weight of dry matter of the *Cannabis sativa* extract.

5. Composition according to the preceding claim, **characterized in that** the *Cannabis sativa* extract comprises between 0.1% and 10% cannabidiol and at least 0.001% terpenes by weight relative to the total weight of dry matter of the *Cannabis sativa* extract.

6. Composition according to the preceding claim, **characterized in that** the terpenes represent at least 0.01% by weight relative to the total weight of dry matter of the *Cannabis sativa* extract.

7. Composition according to the preceding claim, **characterized in that** the terpenes comprise at least myrcenes.

8. Composition according to one of the preceding claims, **characterized in that** the *Crocus sativus* extract comprises

   - at least 2% crocins by weight relative to the total weight of dry matter of the *Crocus sativus* extract, measured by HPLC, and/or
   - flavonoids derived from kaempferol and/or picrocrocin derivatives and/or terpenes, with the exception of safranal.

9. Composition according to one of the preceding claims,
   **characterized in that** the *Crocus sativus* extract is encapsulated with an agent selected from gum arabic, cyclodextrins, fats, vegetable waxes, hydrogenated or non-hydrogenated vegetable oils, hemp oil, proteins, peptides, dextrins, alginates, phospholipids.

10. Composition according to one of the preceding claims, **characterized in that** the composition also comprises an antioxidant agent.

11. Composition according to one of the preceding claims, **characterized in that** the composition comprises at least 15 mg of *Crocus sativus* extract and at least 3 mg of CBD.

12. Composition according to one of the preceding claims,
    **characterized in that** the composition is in dry, liquid or gel form.

13. Composition according to the preceding claim, **characterized in that** it is in the form of a liquid, oily liquid, electronic cigarette liquid, powder, soft capsule, gelcap, tablet, stick, sachet, chewing gum, cream, lotion, oil, gel, transdermal patch, prepared dish or beverage.

14. Composition according to one of the preceding claims for use in preventing and/or treating stress, and/or anxiety, and/or depression, and/or sleep disorders, and/or dementia, and/or cognitive decline, and/or digestive disorders, and/or joint disorders, and/or erectile disorders, and/or vision disorders, and/or disorders related to menopause or related to premenstrual syndrome in humans or animals.

15. Non-therapeutic use of a composition according to one of claims 1 to 13 for improving mood and/or cognitive performance.

Figure 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 3054443

- WO 2017182688 A

**Littérature non-brevet citée dans la description**

- **JACKSON PHILIPPA et al.** Effects of Saffron Extract Supplementation on Mood, Well-Being, and Response to a Psychosocial Stressor in Healthy Adults: A Randomized, Double-Blind, Parallel Group. *Clinical Trial*, 2021

- **GARCIA-RODRIGUEZ et al.** *Comparative evaluation of an ISO 3632 method and an HPLC-DAD method for safranal quantity determination in saffron*, 2017
- **MALLORY E WALTERS et al.** *Potential of Food Hydrolyzed Proteins and Peptides to Chelate Iron or Calcium and Enhance their Absorption*, 2018